# EUROPÄISCHE PATENTSCHRIFT

(11) **EP 3 500 661 B1**
(45) Veröffentlichungstag und Bekanntmachung des Hinweises auf die Patenterteilung: **08.07.2020**
(21) Anmeldenummer: 17754614.0
(22) Anmeldetag: 01.08.2017
(51) Int. Cl.: C12M 3/00, C12M 1/12

(54) **ZELLKULTUREINSATZ UND VORRICHTUNG ZUM KULTIVIEREN VON ZELLEN**
CELL CULTURE INSERT AND DEVICE FOR CULTIVATING CELLS
INSERT DE CULTURE DE CELLULES ET DISPOSITIF DE MISE EN CULTURE DES CELLULES

(30) Priorität: 18.08.2016 DE 202016004992 U; 20.09.2016 DE 202016005740 U
(43) Veröffentlichungstag der Anmeldung: 26.06.2019
(73) Patentinhaber: BRAND GMBH + CO KG, 97877 Wertheim (DE)
(72) Erfinder: FRANK, Nicolas, 97076 Würzburg (DE); ROMAGUERA, Antonio, 97906 Faulbach (DE); PROKOPP, Peter, 97877 Wertheim (DE)
(74) Vertreter: Von Rohr Patentanwälte Partnerschaft mbB
(86) Internationale Anmeldenummer: PCT/EP2017/025225
(87) Internationale Veröffentlichungsnummer: WO 2018/033253

(56) Entgegenhaltungen:
- WO-A1-2008/106515
- WO-A1-2016/069917
- WO-A2-2006/131123
- DE-A1- 10 240 787
- DE-A1-102012 100 583
- DE-T2- 69 109 651
- DE-T2- 69 325 567
- DE-T2- 69 635 398
- FR-A1- 2 942 239
- US-A1- 2006 172 412

## Beschreibung

Die Erfindung betrifft einen Zellkultureinsatz mit den Merkmalen des Oberbegriffs von Anspruch 1 sowie eine Vorrichtung zum Kultivieren von Zellen mit den Merkmalen des Oberbegriffs von Anspruch 12.

Zellkultureinsätze der in Rede stehenden Art werden regelmäßig in sog. Wellplatten verwendet. In der Wellplatte befinden sich mehrere Näpfchen, sog. Wells, zur Aufnahme von Flüssigkeit. Diese sind normalerweise in der Wellplatte eingeformt. Jedes Well weist einen Boden, eine davon aufragende, umlaufende Seitenwand und einen die Seitenwand oben abschließenden Rand auf. Der Rand begrenzt die stirnseitige Zugangsöffnung in das Well.

In die Wells der Wellplatte wird Flüssigkeit gegeben. Dieses ist die Nährflüssigkeit für Zellen, die auf dem Boden des Wells oder auf dem Boden eines in das Well eingesetzten Zellkultureinsatzes gezüchtet werden. Die Nährflüssigkeit wird mittels einer Pipette in das Well eingegeben und gewechselt.

Zellkultureinsätze der in Rede stehenden Art dienen also dazu, Zellen auf dem als Membran ausgeführten Boden der Zellkultureinsätze zu züchten. Zum Einhängen der Zellkultureinsätze in die Wells dienen regelmäßig mehrere am oberen Öffnungsrand des jeweiligen Zellkultureinsatzes seitlich abragende Tragarme. Die Kultivierung von Zellen im hängenden Einsatz in einem relativ großen Abstand zum Boden des Wells erlaubt die Zugabe von ausreichend Nährflüssigkeit in das Well und um den Zellkultureinsatz herum.

Es gibt aber auch Züchtungsmethoden, bei denen die Zellkultureinsätze auf einer Unterlage stehen sollen. Zu diesem Zweck weist der Zellkultureinsatz, von dem die Erfindung ausgeht (US 5,578,492 A), außer den insgesamt vier seitlich abragenden Tragarmen am oberen Öffnungsrand bodenseitig am Rand des Gehäuses insgesamt sechs nach unten abragende Stützfüßchen auf. Mit diesen kann das Gehäuse dieses Zellkultureinsatzes auf einer Unterlage mit geringem, gleichmäßigem Abstand seines Bodens von der Unterlage kippsicher abgestellt werden.

Der Boden des Zellkultureinsatzes ist regelmäßig als eine mikroporöse Membran ausgeführt. Die Membran ermöglicht einen Austausch mit der Nährflüssigkeit unter dem Boden des Einsatzes, wenn der Zellkultureinsatz in einem mit Nährflüssigkeit gefüllten Well einer Wellplatte angeordnet ist.

Bei dem Stand der Technik, von dem die Erfindung ausgeht (US 5,578,492 A), besteht der Zellkultureinsatz aus einem thermoplastischen Kunststoff, beispielsweise PE, PET, PP, der an den Tragarmen gehäuseseitig mit Sollbruchstellen versehen ist. Man kann die Tragarme also vom Gehäuse des Zellkultureinsatzes abbrechen, um den Zellkultureinsatz nicht mehr am umlaufenden Rand des Wells der Wellplatte aufzuhängen, sondern auf seinen Stützfüßchen am gewünschten Ort, beispielsweise auch im Well, abzustellen, wobei der Boden des Zellkultureinsatzes auch dann immer noch einen bestimmten Abstand von der Unterlage, in dem Fall dem Boden des Wells, einhält.

Im zuvor beschriebenen Stand der Technik werden Methoden erläutert, wie ein solcher Zellkultureinsatz hergestellt werden kann, insbesondere aus welchem Material der als Membran ausgeführte Boden des Zellkultureinsatzes bestehen und wie dieser Boden an dem Gehäuse des Zellkultureinsatzes angebracht werden kann. Für diese Informationen aus dem Stand der Technik wird auf diesen Stand der Technik verwiesen. Derartige Konstruktionen des Zellkultureinsatzes sind auch im Rahmen der Lehre der Erfindung bevorzugt anwendbar.

Zum Einbringen von Nährflüssigkeit in das Well oder zum Entnehmen von Nährflüssigkeit aus dem Well mit Hilfe der Pipettenspitze einer Pipette benötigt man ein wenig Platz seitlich neben dem im Well eingesetzten Zellkultureinsatz. Man benötigt also mindestens ein sog. Beschickungsfenster zwischen Zellkultureinsatz und Seitenwand des Wells. Dieses Beschickungsfenster ist bei dem im Stand der Technik, von dem die Erfindung ausgeht, vorgesehenen Zellkultureinsatz vergleichsweise eng.

Auch bei anderen Konstruktionen des Standes der Technik sind die Beschickungsfenster vergleichsweise eng und daher fehleranfällig (DE 10 2012 100 583 A1; US 2006/0172412 A1; DE 693 25 567 T2). Man versucht das mit einer exzentrischen Anordnung des Zellkultureinsatzes im Well zu lösen, jeweils wieder mit einer Mehrzahl von gleichmäßig auf dem Umfang verteilten Tragarmen (EP 1 532 237 B1). Andere Ansätze versuchen es mit einer speziellen Anpassung von Wellplatte und Zellkultureinsätzen, wobei in der Wellplatte dann asymmetrische Zusatzausnehmungen als Beschickungsfenster vorgesehen sind (US 5,534,227 A; WO 2006/131123 A2; FR 2 942 239 A1). Derartige Konstruktionen sind aufwendig.

In dem Dokument WO 2016/069917 A1 werden dreidimensionale Zellenverbunde direkt auf dem Boden des Wells gezüchtet. Ein Einsatz mit gitterförmigem Boden ist für Flüssigkeit und einzelne Zellen durchlässig, hält jedoch den Zellenverbund am Wellboden.

Der Lehre liegt das Problem zugrunde, einen Zellkultureinsatz zu schaffen, der hängend und stehend einsetzbar ist und gleichwohl auf einfache Weise ein ausreichendes Beschickungsfenster im Well gewährleistet. Aufgabe der Erfindung ist auch die Angabe einer entsprechenden Vorrichtung zum Kultivieren von Zellen mit Wellplatte und Zellkultureinsatz.

Das zuvor aufgezeigte Problem ist bei einem Zellkultureinsatz mit den Merkmalen des Oberbegriffs von Anspruch 1 durch die Merkmale des kennzeichnenden Teils von Anspruch 1 gelöst. Bevorzugte Ausgestaltungen und Weiterbildungen sind Gegenstand der auf den Zellkultureinsatz gerichteten Unteransprüche.

Erfindungsgemäß ist der Zellkultureinsatz mit einem speziell ausgestalteten Tragarm versehen, der nämlich als Rand-Einhängeelement ausgeführt ist. Somit reicht ein einziger nach außen abragender Tragarm, um den Zellkultureinsatz sicher am Rand eines Wells einer Wellplatte einzuhängen. Auf diese Weise benötigt man auch nicht mehrere nach außen abragende Tragarme, sondern im Grundsatz nur genau einen Tragarm, der dieses leistet.

Bei entsprechender Bemaßung des Gehäuses des Zellkultureinsatzes im Vergleich mit dem zu dessen Aufnahme bestimmten Well der Wellplatte hat man so bei eingehängtem Zellkultureinsatz auf der dem eingehängten Tragarm gegenüberliegenden Seite des Gehäuses des Zellkultureinsatzes einen wesentlich größeren Abstand von der Seitenwand des Wells als auf der dem Tragarm zugewandten Seite des Gehäuses. Auf der dem Tragarm gegenüberliegenden Seite des Gehäuses schafft man so zwanglos das gewünschte Beschickungsfenster für das Well.

Im Grundsatz reicht, wie bereits erläutert, ein einziger als Rand-Einhängeelement ausgeführter Tragarm am Gehäuse. Grundsätzlich ist es allerdings auch im Rahmen der Lehre der Erfindung möglich, mehr als einen als Rand-Einhängeelement ausgeführten Tragarm am Gehäuse vorzusehen. Man könnte also beispielsweise auch im Rahmen der Lehre der Erfindung so, wie das bereits im Stand der Technik verwirklicht worden ist, drei um jeweils ca. 120° auf dem Umfang des Öffnungsrandes des Gehäuses verteilt angeordnete Tragarme vorsehen, die hier nun jeweils als Rand-Einhängeelement ausgeführt sind.

Besonders zweckmäßig ist allerdings der erfindungsgemäße Zellkultureinsatz dann ausgestaltet, wenn er genau einen als Rand-Einhängeelement ausgeführten Tragarm aufweist.

Wesentlich ist in jedem Fall, dass die Außenhöhe des Zellkultureinsatzes geringer ist als die Innenhöhe des Wells, in das der Zellkultureinsatz eingesetzt werden soll, um somit zu erreichen, dass bei eingehängtem Zellkultureinsatz sich die Stützfüßchen des Zellkultureinsatzes in einem Abstand vom Boden des Wells befinden.

Je nach den lateralen Innenabmessungen des Wells, mit dem der erfindungsgemäße Zellkultureinsatz eingesetzt werden soll, kann man den Zellkultureinsatz mitsamt dem Tragarm, der dann aber nicht genutzt wird, mittels der Stützfüßchen auf dem Boden des Wells der Wellplatte abstellen. Auf diese Weise ergibt sich seitlich des Tragarms im Well von selbst jeweils ein Beschickungsfenster für das Well, weil der Tragarm am Öffnungsrand des Zellkultureinsatzes nach außen abragt und so einen Abstand zur Seitenwand des Wells gewährleistet.

Es kann aber auch so sein, dass die lateralen Innenabmessungen des Wells, mit dem der Zellkultureinsatz angewendet werden soll, einen Einsatz des Zellkultureinsatzes in das Well mit Tragarm nicht erlaubt. In diesem Fall müsste man, wie an sich aus dem Stand der Technik bekannt, den Tragarm gehäusenah vom Gehäuse des Zellkultureinsatzes abbrechen, um den Zellkultureinsatz dann im Well auf dem Boden abstellen zu können.

Sind am Zellkultureinsatz mehrere auf den Umfang des Öffnungsrandes des Gehäuses verteilt angeordnete Tragarme vorgesehen, so müssen dann, wenn man den Zellkultureinsatz auf dem Boden des Wells abstellen können möchte, die lateralen Innenabmessungen des Wells größer sein als die lateralen Außenabmessungen des Zellkultureinsatzes mit Berücksichtigung der Tragarme. In diesem Fall kann man trotz Vorhandensein mehrerer Tragarme den Zellkultureinsatz immer nur mit einem als Rand-Einhängeelement ausgeführten Tragarm am Well einhängen.

Will man hingegen bei mehreren auf dem Umfang des Öffnungsrandes des Gehäuses verteilt angeordneten Tragarmen zum einen den Zellkultureinsatz mit allen Tragarmen gleichzeitig am Well einhängen können und gleichwohl ein Abstellen des Zellkultureinsatzes auf dem Boden des Wells erlauben, so bedarf es einer besonderen Gestaltung des Wells. Dazu ist es dann nämlich erforderlich, dass die laterale geometrische Form des Wells asymmetrisch relativ zur lateralen geometrischen Form des Gehäuses des Zellkultureinsatzes ausgebildet ist. Bei drei Tragarmen müsste das Well nach Art eines Dreiecks ausgeführt sein. In der Anordnung der Tragarme im Schnittpunkt am Inkreis des Wells könnte der Zellkultureinsatz am Rand des Wells eingehängt werden. In der Anordnung der Tragarme ausgerichtet auf den Umkreis des Wells könnte der Zellkultureinsatz auf dem Boden des Wells abgestellt werden.

In jedem Fall wird erfindungsgemäß erreicht, dass mit dem Zellkultureinsatz Zellkulturen hängend kultiviert und stehend kultiviert und/oder verschickt werden können. Bei hängender Anordnung des Zellkultureinsatzes lässt sich ein relativ großer Abstand der Membran des Zellkultureinsatzes zum Boden des Wells vorgeben. Dadurch entsteht ein verhältnismäßig großes Volumen im Well unterhalb der Membran des Zellkultureinsatzes. Dadurch steht Zell- und Gewebekulturen, die an einer Luft-Flüssigkeits-Grenzschicht gezüchtet werden müssen, mehr Nährmedium zur Verfügung. Ein solcher Zellkultureinsatz kann einschließlich der darin gezüchteten Kultur anschließend auf den Boden des Wells einer üblichen Wellplatte gestellt und, fixiert durch ein geeignetes Einbettungsmedium sowie geschützt durch einen Deckel, transportiert werden.

Nach bevorzugter Lehre der Erfindung empfiehlt es sich, dass der lichte Abstand des Einhängeabschnittes von dem Gehäuse größer ist als die Dicke des Randes eines Wells, so dass sich für den eingesetzten Zellkultureinsatz im Well ein Spielsitz ergibt. Damit lässt sich der Zellkultureinsatz im Well leicht aufwärts und abwärts bewegen, also einhängen und auch wieder entnehmen.

Man kann durch Gestaltung des Tragarms des erfindungsgemäßen Zellkultureinsatzes eine bestimmte Lage des Zellkultureinsatzes im Well vorgeben. Dazu kann man vorsehen, dass der Tragarm einen vom Gehäuse aus nach oben verlaufenden Anfangsabschnitt aufweist, an den sich der Auflageabschnitt anschließt, so dass bei horizontal ausgerichtetem Gehäuse der untere Rand des Auflageabschnitts höher liegt als der Öffnungsrand.

Der Anfangsabschnitt kann beispielsweise zunächst vom Öffnungsrand leicht nach außen und oben verlaufen und dann in den Auflageabschnitt übergehen. Eingehängt im Well befindet sich dann der Öffnungsrand des Zellkultureinsatzes in einer Ebene deutlich unterhalb der Ebene, die durch den Rand des Wells gebildet ist.

Die Flexibilität des erfindungsgemäßen Zellkultureinsatzes wird dadurch erhöht, dass der Tragarm gehäusenah, vorzugsweise im Anfangsabschnitt oder im Auflageabschnitt, eine Sollbruchstelle aufweist, an der der Tragarm vom Gehäuse abbrechbar ist. Die Sollbruchstelle ist vorzugsweise kreisbogenförmig ausgeführt. Sie ist dadurch weniger biegeelastisch. Man kann also den Zellkultureinsatz mit Tragarm, später bei der Verwendung aber auch ohne Tragarm einsetzen. Damit hat der erfindungsgemäße Zellkultureinsatz eine große Anwendungsbreite für unterschiedliche Wells und unterschiedliche Anwendungszwecke.

Es ist immer wieder schwierig, einen Zellkultureinsatz fehlerfrei mit einem Greifwerkzeug, insbesondere mit einer Pinzette, zu handhaben. Insbesondere darf sich der Zellkultureinsatz unter Einfluss der Kraftausübung durch das Greifwerkzeug nicht drehen oder neigen. Hier bietet erfindungsgemäß der Einhängeabschnitt des Tragarms bei entsprechender Gestaltung eine vorzügliche Hilfe. Der Einhängeabschnitt ist stegartig gestaltet und kann von einer Pinzette erfasst werden. Bei entsprechender Gestaltung des Einhängeabschnittes, beispielsweise dadurch, dass an seinem oberen Rand beidseitig eine Anformung vorgesehen ist, kann die Spitze der Pinzette zwischen diesen Anformungen angreifen. Der Zellkultureinsatz kann sich so gegenüber der Spitze der Pinzette nicht drehen, sondern kann vielmehr lagestabil aus dem Well entnommen oder in das Well eingehängt werden.

Ganz besonders zweckmäßig ist eine Konstruktion des erfindungsgemäßen Zellkultureinsatzes, bei der am Gehäuse genau ein Tragarm vorgesehen ist. Damit hängt der Zellkultureinsatz im Well exzentrisch und der Bereich zwischen Gehäuse des Zellkultureinsatzes und Seitenwand des Wells jenseits des Tragarms ist völlig frei. Hier ergibt sich bei eingehängtem Zellkultureinsatz ein breites Beschickungsfenster für eine Pipettenspitze.

Das mit dem Tragarm im Well eingehängte Gehäuse des Zellkultureinsatzes hängt aufgrund des Spielsitzes am Rand des Wells leicht geneigt gegenüber dem Well. Die Lage des eingehängten Zellkultureinsatzes kann man dadurch stabilisieren, dass man unter Nutzung des Einhängeabschnitts des Tragarms und der Stützfüßchen eine Dreipunkt-Lagerung des Gehäuses an der Seitenwand des Wells generiert. Um das zu erreichen, ist nach bevorzugter Lehre der Erfindung vorgesehen, dass zwei vom Gehäuse auch nach außen abragende Stützfüßchen auf dem Umfang des Gehäuses beidseitig und mit gleichem Abstand zum Tragarm angeordnet sind. Mittels dieser beiden, sich an der Seitenwand des Wells abstützenden Füßchen lässt sich erreichen, dass ein im Well eingehängter Zellkultureinsatz gegenüber dem Well um nicht mehr als 10° geneigt angeordnet ist.

Damit gleichwohl der Zellkultureinsatz dann, wenn er auf einer Unterlage abzustellen ist, sicher und kippsicher steht, ist nach bevorzugter Lehre der Erfindung vorgesehen, dass genau drei auf dem Umfang des Gehäuses gleich beabstandete Stützfüßchen vorgesehen sind.

Wenn nach bevorzugter Lehre die Stützfüßchen vom Gehäuse des Zellkultureinsatzes auch nach außen abragen, hat das den Vorteil, dass zwischen der Seitenwand des Wells und dem Gehäuse bei im Well eingesetztem Zellkultureinsatz ein Spalt beibehalten wird. Das verbessert den Austausch von Nährflüssigkeit. Zudem kann so die den Boden des hohlzylindrischen Gehäuses bildende Membran am unteren umlaufenden Rand des hohlzylindrischen Gehäuses befestigt sein, so dass die maximale Fläche der Membran genutzt werden kann. Wenn man den von den Stützfüßchen nach außen erzeugten Abstand hinreichend groß bemisst, kann man bei in Nährflüssigkeit eingetauchtem Zellkultureinsatz einen Kapillareffekt vermeiden oder jedenfalls so begrenzen, dass das Flüssigkeitsniveau im Well überall im Wesentlichen gleichhoch ist. Dadurch wird die Membran stets vollflächig mit Nährflüssigkeit benetzt und das Entstehen von nicht oder nur schlecht versorgten Bereichen der Membran kann verhindert werden.

Ganz besonders bevorzugt ist eine Konstruktion, bei der das Gehäuse des Zellkultureinsatzes kreiszylindrisch ausgeführt ist, wobei dann besonders bevorzugt entsprechend auch das zugeordnete Well der Wellplatte kreiszylindrisch ausgeführt sein sollte.

Werkstofftechnisch empfiehlt es sich, dass der Zellkultureinsatz aus Kunststoff, insbesondere aus einem thermoplastischen Kunststoff, besteht. Hier darf wieder auf den Stand der Technik verwiesen werden, in dem Vorschläge für sinnvoll zu verwendende Werkstoffe gemacht werden.

Gegenstand der Erfindung ist auch eine Vorrichtung zum Kultivieren von Zellen mit den Merkmalen des Oberbegriffs von Anspruch 12. Bei dieser ist die zuvor aufgezeigte Aufgabe durch die Merkmale des kennzeichnenden Teils von Anspruch 12 gelöst.

Die Besonderheiten der Anordnung eines Zellkultureinsatzes gemäß der Erfindung in ein Well einer Vorrichtung zum Kultivieren von Zellen sind im Zusammenhang mit der Erläuterung des Zellkultureinsatzes oben bereits dargestellt worden. Auf diese Ausführungen darf verwiesen werden.

Für die Wellplatte gilt ebenfalls, dass sie aus Kunststoff, insbesondere aus einem thermoplastischen Kunststoff, bestehen kann. Sie ist zumeist mit einer zusätzlichen Bodenplatte oder einem Bodenrahmen kombiniert und hat häufig auch einen Deckel. Auch insoweit darf auf den eingangs erläuterten Stand der Technik verwiesen werden.

Im Folgenden wird die Erfindung nun anhand einer lediglich ein bevorzugtes Ausführungsbeispiel darstellenden Zeichnung näher erläutert. In der Zeichnung zeigt
- Fig. 1: in perspektivischer Darstellung eine erfindungsgemäße Vorrichtung zum Kultivieren von Zellen mit einer Wellplatte mit mehreren, hier insgesamt sechs Wells zur Aufnahme von Flüssigkeit, und mit mehreren, hier insgesamt vier Zellkultureinsätzen,
- Fig. 2: die Vorrichtung aus Fig. 1 in einer Draufsicht,
- Fig. 3: die Vorrichtung aus Fig. 2 im Schnitt gemäß der Linie III-III in Fig. 2,
- Fig. 4: die Vorrichtung aus Fig. 2 im Schnitt gemäß der Linie IV-IV in Fig. 2,
- Fig. 5: in perspektivischer Ansicht schräg von oben einen erfindungsgemäßen Zellkultureinsatz und
- Fig. 6: in einer perspektivischen Ansicht schräg von unten den Zellkultureinsatz aus Fig. 5.

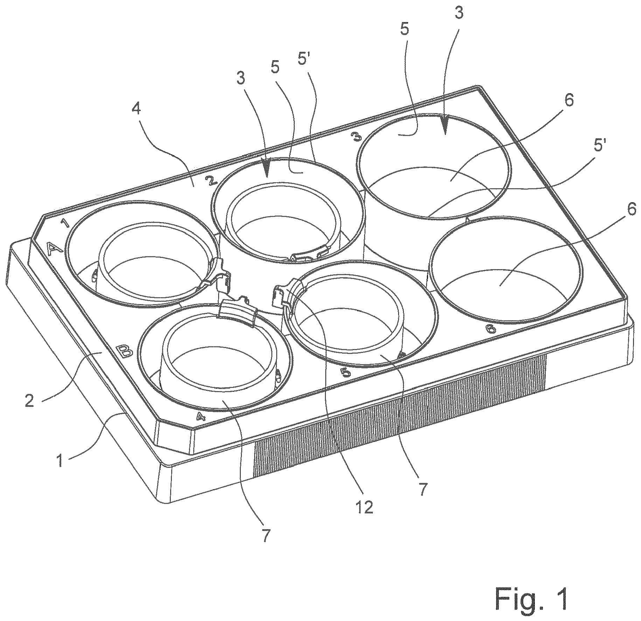

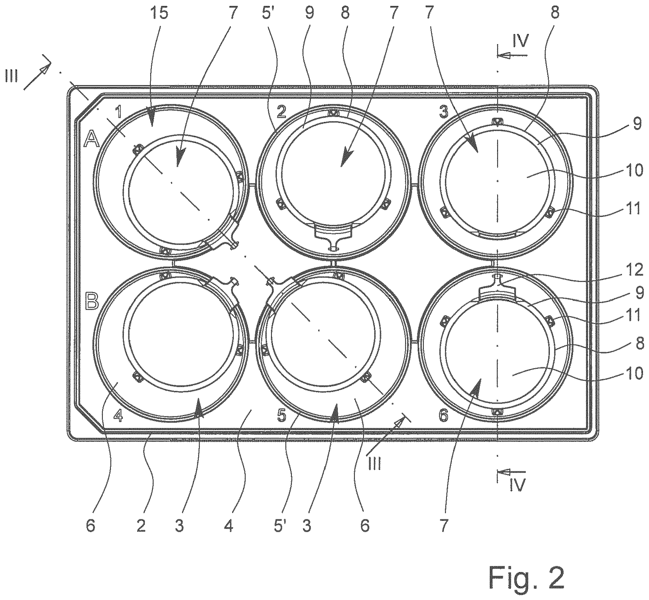

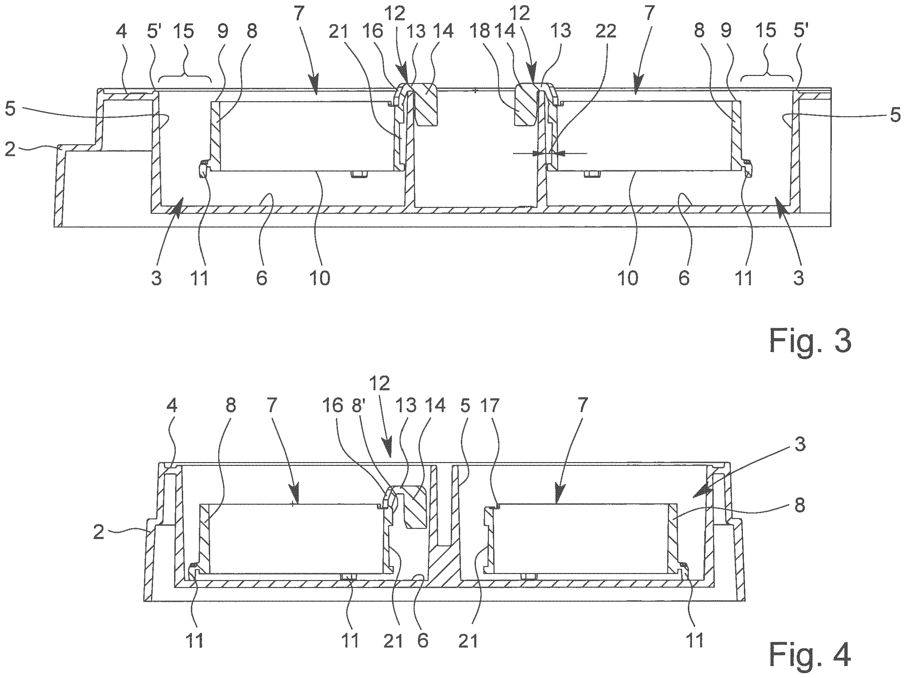

Fig. 1 und 2 zeigen im Überblick, Fig. 3 und 4 jeweils im Schnitt, ein besonders bevorzugtes Ausführungsbeispiel einer Vorrichtung zum Kultivieren von Zellen. Diese weist eine hier mit einer Stufe 1 versehene Wellplatte 2 auf, die aus thermoplastischem Kunststoff ausgeführt ist. In der Wellplatte 2 befinden sich mehrere, hier insgesamt sechs, Wells 3 zur Aufnahme von Flüssigkeit. Die Wellplatte 2 hat eine sich über einen Teil der Fläche erstreckende Oberseite 4, auf der sich in Fig. 1 die Identifikations-Ziffern befinden und in die Wells 3 integral eingeformt sind. In den Zwischenräumen zwischen den Wells 3 ist die Oberseite 4 vertieft, so dass man dort die Seitenwände 5 der Wells 3 sehen kann, weil diese frei zugänglich sind.

In Fig. 1 sieht man bereits, dass jedes Well 3 einen Boden 6 und die davon aufragende, umlaufende Seitenwand 5 aufweist, wobei die Seitenwand 5 einen diese oben abschließenden Rand 5' aufweist. In Fig. 1 kann man rechts an den leeren Wells 3 gut nachvollziehen, dass jedes Well 3 in einer Ebene parallel zum Boden 6 bestimmte laterale Innenabmessungen und in Richtung senkrecht zum Boden 6 eine bestimmte Innenhöhe aufweist. Im dargestellten Ausführungsbeispiel sind die Wells 3 zylindrisch ausgeführt, so dass die lateralen Innenabmessungen durch einen bestimmten Innendurchmesser abschließend definiert sind. Bei nichtkreisrunden Wells 3 gibt es komplexere laterale Innenabmessungen.

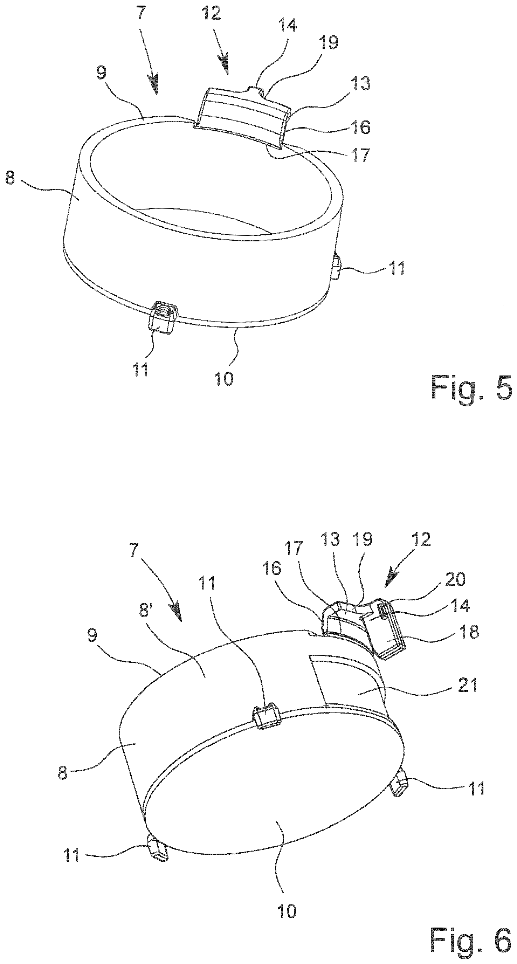

Fig. 1 zeigt in den Wells 3 mit der Nummerierung 1, 2, 4, 5 jeweils einen Zellkultureinsatz 7. In Fig. 2 sind nicht nur in den vier genannten Wells 3 Zellkultureinsätze 7 vorhanden, sondern auch in den ganz rechts befindlichen Wells mit den Nummern 3 und 6 befinden sich Zellkultureinsätze 7. In Fig. 3 und 4 sind die in Fig. 2 eingezeichneten Schnitte der Vorrichtung dargestellt. Fig. 5 und 6 zeigen einen bevorzugten erfindungsgemäßen Zellkultureinsatz 7 in zwei unterschiedlichen perspektivischen Darstellungen.

Nachfolgend wird auf alle Figuren im Zusammenhang Bezug genommen.

Der erfindungsgemäße Zellkultureinsatz 7 ist ein solcher mit einem hohlzylindrischen Gehäuse 8 mit einer stirnseitigen oberen, durch einen Rand 9 begrenzten Öffnung und einem stirnseitigen unteren, als Membran ausgeführten Boden 10, mit am bodenseitigen Rand des Gehäuses 8 und/oder am Boden 10 angeordneten, nach unten abragenden Stützfüßchen 11, mit denen das Gehäuse 8 auf einer Unterlage mit geringem, gleichmäßigem Abstand des Bodens 10 von der Unterlage kippsicher abstellbar ist, und mit mindestens einem vom Gehäuse 8 am Öffnungsrand 9 nach außen abragenden Tragarm 12, der auf einem Rand 5' eines Wells 3 einer Wellplatte 2 auflegbar ist.

Im dargestellten Ausführungsbeispiel, besonders gut erkennbar in dem Schnitt von Fig. 4, befinden sich die Stützfüßchen 11 am bodenseitigen Rand des Gehäuses 8 und nicht am Boden 10. Sie können in anderen Ausführungsformen aber auch unmittelbar am Boden 10 oder im Übergang zwischen Gehäuse 8 und Boden 10 angeordnet sein.

Fig. 4 kann man ferner besonders gut entnehmen, dass der Boden 10 als dünne permeable Membran ausgeführt ist. Dies entspricht der typischen Konstruktion von Zellkultureinsätzen 7. Hier können entsprechende Vorschläge aus dem eingangs erläuterten Stand der Technik Anwendung finden, insbesondere hinsichtlich der Auswahl der Werkstoffe für den Boden 10 und der Art und Weise der Anbringung des Bodens 10 am Gehäuse 8 des Zellkultureinsatzes 7.

Aus Fig. 3, Fig. 4 und insbesondere Fig. 6 lässt sich entnehmen, dass bei dem erfindungsgemäßen Zellkultureinsatz 7 vorgesehen ist, dass der Tragarm 12 als Rand-Einhängeelement ausgeführt ist und dazu einen radial zum Gehäuse 8 verlaufenden Auflageabschnitt 13 und einen vom Auflageabschnitt 13 nach unten in Richtung des Bodens 10 des Gehäuses 8 verlaufenden Einhängeabschnitt 14 aufweist. "Radial" ist bei einem kreiszylindrischen Gehäuse 8 eine eindeutige Angabe, bei einem nicht kreiszylindrischen Gehäuse 8 bedeutet sie die Ausrichtung vom Rand des Gehäuses 8 nach außen.

Man sieht in Fig. 3, wie der erfindungsgemäße Zellkultureinsatz 7 mit dem Tragarm 12 problemlos locker in das Well 3 eingehängt werden kann. Dabei sieht man gut, dass sich der Öffnungsrand 9 des Zellkultureinsatzes 7 in dem Well 3, unterhalb des Randes 5' des Wells 3 befindet. Bei dem eingehängten Zellkultureinsatz 7 befinden sich die Stützfüßchen 11 in einem Abstand vom Boden 6 des Wells 3. Die Außenhöhe des Zellkultureinsatzes ist also geringer als die Innenhöhe des Wells 3.

Aus Fig. 2, Fig. 3 und Fig. 4 lässt sich entnehmen, dass im dargestellten Ausführungsbeispiel entsprechend einer bevorzugten Lehre der Erfindung der Zellkultureinsatz 7 bestimmte laterale Außenabmessungen aufweist und dass die lateralen Außenabmessungen des Zellkultureinsatzes 7 ohne Berücksichtigung des mindestens einen Tragarmes 12 geringer sind als die lateralen Innenabmessungen des Wells 3, und zwar dergestalt, dass auf der dem eingehängten Tragarm 12 gegenüberliegenden Seite das Gehäuse 8 des Zellkultureinsatzes 7 einen wesentlich größeren Abstand von der Seitenwand 5 des Wells 3 aufweist als auf der dem Tragarm 12 zugewandten Seite des Gehäuses 8. Man sieht in Fig. 3, dass sich gegenüber dem jeweiligen Tragarm 12 im Well 3 ein großes Beschickungsfenster 15 findet, durch das Nährflüssigkeit in das Well 3 eingegeben oder aus dem Well 3 mittels einer Pipettenspitze entnommen werden kann. Entsprechend sieht man die Beschickungsfenster 15 auch gut in Fig. 1 und 2.

In Fig. 4 sieht man im Vergleich des links im Well 3 und rechts im benachbarten Well 3 befindlichen Zellkultureinsatzes 7, dass die lateralen Außenabmessungen des Zellkultureinsatzes 7 mit Berücksichtigung des mindestens einen Tragarms 12 geringer sind als die lateralen Innenabmessungen des Wells 3 und dass somit der Zellkultureinsatz 7 mitsamt dem mindestens einen Tragarm 12 auf dem Boden 6 des Wells 3 abstellbar ist. Die Lage des Schnittes sieht man in Fig. 2. Man sieht in Fig. 2 im rechts unten liegenden Well 3 mit der Nummer 6 oben rechts und links neben dem Einhängeabschnitt 14 des dortigen Tragarms 12 einen größeren Abstand zur Seitenwand 5 des Wells 3 als auf der vom Tragarm 12 abgewandten Seite des Gehäuses 8. Der Abstand rechts und links vom Tragarm 12 generiert jeweils ein auch noch ausreichendes Beschickungsfenster 15.

Fig. 3 lässt bei den eingehängten Zellkultureinsätzen 7 erkennen, dass der lichte Abstand des Einhängeabschnittes 14 von dem Gehäuse 8 größer ist als die Dicke des Randes 5' eines Wells 3, so dass sich für den eingesetzten Zellkultureinsatz 7 im Well 3 ein Spielsitz ergibt. Das ist anhand der dargestellten Spalte gut nachvollziehbar.

Das in Fig. 5 und 6 dargestellte bevorzugte Ausführungsbeispiel eines Zellkultureinsatzes 7 zeigt weiter eine konstruktive Besonderheit dergestalt, dass der Tragarm 12 einen vom Gehäuse 8 aus nach oben verlaufenden Anfangsabschnitt 16 aufweist, an den sich der Auflageabschnitt 13 anschließt, und zwar dergestalt, dass bei im Well 3 eingehängtem Zellkultureinsatz 7 der Öffnungsrand 9 unterhalb des Randes 5' des Wells 3 liegt. Das hat im Ergebnis die Lage des Öffnungsrandes 9 des Zellkultureinsatzes 7 leicht unterhalb des Randes 5' der Seitenwand 5 des Wells 3 zur Folge, wie in Fig. 3 der Zeichnung links und rechts gut zu erkennen ist. Durch die Ausgestaltung des Tragarms 12 in den verschiedenen Abschnitten wird so die vertikale Position des Zellkultureinsatzes 7 im Well 3 der Wellplatte 2 vorgegeben.

Fig. 6 lässt rechts oben am Tragarm 12 als eine weitere Besonderheit des erfindungsgemäßen Zellkultureinsatzes 7 erkennen, dass der Tragarm 12 gehäusenah, hier und vorzugsweise im Anfangsabschnitt 16, eine Sollbruchstelle 17 aufweist, an der der Tragarm 12 vom Gehäuse 8 abbrechbar ist, wobei die Sollbruchstelle 17 vorzugsweise kreisbogenförmig ausgeführt und dadurch recht biegestabil ist. Eine Sollbruchstelle 17 ist auch bereits im Stand der Technik zu Tragarmen 12 der in Rede stehenden Art empfohlen worden. Sie verläuft hier kreisbogenförmig am Gehäuse 8, weil das Gehäuse 8 des Zellkultureinsatzes 7 hier insgesamt kreiszylindrisch ausgeführt ist.

In Fig. 4 sieht man rechts einen Zellkultureinsatz 7 ohne den dort bereits abgebrochenen Tragarm 12 und erkennt, dass hier viel Platz rechts und links des Zellkultureinsatzes 7 im Well 3 vorliegt.

Fig. 1 und 6 lassen im Zusammenhang eine weitere Besonderheit des erfindungsgemäßen Zellkultureinsatzes 7 erkennen, die mit der Handhabbarkeit des Zellkultureinsatzes 7 mittels einer Pinzette zu tun hat. Es ist nämlich bei dieser Konstruktion vorgesehen, dass der Einhängeabschnitt 14 des Tragarms 12 nach außen verlängert als Griffsteg 18 für ein Greifwerkzeug, insbesondere eine Pinzette, ausgeführt ist. Die Pinzette, die hier nicht dargestellt ist, kann rechts und links an den Flächen des Griffstegs 18 angreifen. Sie rutscht aber von dem Griffsteg 18 nicht ab, weil rechts und links bogenartige Ausformungen 19 und in diesem Beispiel auch beidseitig am Griffsteg 18 erhabene Anformungen 20 die Schenkel der Pinzette formschlüssig umranden und so halten und führen. Somit kann mit dieser Konstruktion der Zellkultureinsatz 7 mittels einer Pinzette oder eines anderen Greifwerkzeugs lagesicher transportiert werden.

Der in Fig. 5 und 6 in zwei perspektivischen Ansichten in vergrößerter Darstellung gezeigte Zellkultureinsatz 7 ist ganz besonders zweckmäßig ausgeführt, er weist nämlich am Gehäuse 8 nicht mehrere Tragarme 12, sondern genau einen Tragarm 12 auf. Dieser dient dem Einhängen des Zellkultureinsatzes 7 am Rand 5' des Wells 3. Das reicht aus, mehr braucht man für den erfindungsgemäßen Zellkultureinsatz 7 nicht. Das ist konstruktiv einfach und spart Material. Durch den Einhängeabschnitt 14 erfüllt der Tragarm 12 erfindungsgemäß die Funktion, die im Stand der Technik durch mehrere Tragarme 12 am Gehäuse 8 des Zellkultureinsatzes 7 des Standes der Technik verwirklicht wird.

Die Figuren lassen ferner eine Besonderheit der erfindungsgemäßen Konstruktion erkennen, die sich dadurch auszeichnet, dass die Stützfüßchen 11 vom Gehäuse 8 auch nach außen abragen, wobei zwei Stützfüßchen 11 auf dem Umfang des Gehäuses 8 zum Tragarm 12 seitlich gleich beabstandet angeordnet sind. Auf diese Weise wirken die Stützfüßchen 11 bei eingehängtem Zellkultureinsatz 7 mit dem Tragarm 12 dahingehend zusammen, dass sich insgesamt eine Dreipunkt-Lagerung des Gehäuses 8 des Zellkultureinsatzes 7 an der Seitenwand 5 des Wells 3 ergibt (Tragarm 12, erstes Stützfüßchen 11, zweites Stützfüßchen 11). Man kann das anhand von Fig. 6 und Fig. 3 gut nachvollziehen.

Bei richtiger Anordnung der verschiedenen Komponenten kann man erreichen, dass die Anordnung des Einhängeabschnittes 14 des Tragarms 12 einerseits und der dem Tragarm 12 zugeordneten Stützfüßchen 11 andererseits so gewählt ist, dass ein im Well 3 eingehängter Zellkultureinsatz 7 gegenüber dem Well 3 um nicht mehr als 10° geneigt angeordnet ist. Dies sieht man in der Darstellung in Fig. 3, der Neigungswinkel beträgt hier etwa 3°.

Der in Fig. 6 dargestellte Zellkultureinsatz 7 zeigt hier und bevorzugt am Außenumfang 8' des Gehäuses 8 gegenüber dem Tragarm 12 eine Einbuchtung 21. Diese erstreckt sich am Außenumfang 8' symmetrisch zum Tragarm 12 und längs des Gehäuses 8. Die Einbuchtung 21 ist angrenzend zum Boden 10 ausgeformt und ist mindestens 50% der Außenhöhe des Zellkultureinsatzes 7 hoch. Die Position und Höhe der Einbuchtung 21 ist dem Niveau von im Well 3 eingefüllter Flüssigkeit angepasst. Das Niveau liegt stets über der Höhe des Bodens 10 eines im Well 3 eingehängten oder auf den Stützfüßchen 11 abgestellten Zellkultureinsatzes 7.

Wie in Fig. 3 gezeigt, ist der Abstand zwischen der Seitenwand 5 und dem Außenumfang 8' des eingehängten Zellkultureinsatzes 7 gering bis gleich Null. Die Einbuchtung 21 unterbricht eine im kritischen Bereich zwischen der Seitenwand 5 und dem Außenumfang 8' entstehende Kapillarität der Flüssigkeit im Well 3. Die Einbuchtung 21 ist am Außenumfang 8' entsprechend dimensioniert und stellt sicher, dass die eingefüllte Flüssigkeit auch in diesem kritischen Bereich nicht wesentlich höher als deren Niveau im Well 3 steigt. Das von der Kapillarität beeinflusste Niveau soll zumindest innerhalb der Höhe der Einbuchtung 21 verbleiben.

Die Einbuchtung 21 kann bezogen zum Außenumfang 8' und wie in Fig. 6 gezeigt gleichförmig oder unterschiedlich tief sein. Die Tiefe im Ausführungsbeispiel ist so gewählt, dass in dem kritischen Bereich ein Abstand 22 zwischen der Einbuchtung 21 des eingehängten Zellkultureinsatzes 7 und der Seitenwand 5 1 mm nicht unterschreitet. Die Kapillarität wird beeinflusst von der Flüssigkeitsdichte, der Oberflächenspannung der Flüssigkeit am Außenumfang 8' bzw. der Einbuchtung 21 und der Seitenwand 5 und vom Abstand 22.

Im dargestellten und bevorzugten Ausführungsbeispiel ist durchweg vorgesehen, dass die Gehäuse 8 der Zellkultureinsätze 7 kreiszylindrisch ausgeführt sind. Das ist besonders zweckmäßig, allerdings nicht zwingend. Andere laterale Querschnitte von Zellkultureinsätzen sind im Stand der Technik ebenso beschrieben wie entsprechende Querschnitte von Wells entsprechender Wellplatten. Erfindungsgemäß ist es allerdings besonders zweckmäßig, wenn sowohl die Wells 3 der Wellplatte 2 als auch die Gehäuse 8 der Zellkultureinsätze 7 kreiszylindrisch ausgeführt sind.

Für die Zellkultureinsätze 7 wie auch für die Wellplatte 2 gilt, dass in bevorzugter Weise Kunststoff, insbesondere ein thermoplastischer Kunststoff, Verwendung findet. Beispiele hierzu sind einerseits einleitend angegeben worden, finden sich andererseits umfangreich im Stand der Technik.

Alle oben geschilderten Aspekte der Erfindung sind sowohl einzeln als auch in Kombination miteinander anwendbar.

### Bezugszeichenliste:

- 1: Stufe
- 2: Wellplatte
- 3: Well
- 4: Oberseite
- 5: Seitenwand
- 5': Rand von 5
- 6: Boden
- 7: Zellkultureinsatz
- 8: Gehäuse
- 8': Außenumfang
- 9: Öffnungsrand
- 10: Boden
- 11: Stützfüßchen
- 12: Tragarm
- 13: Auflageabschnitt
- 14: Einhängeabschnitt
- 15: Beschickungsfenster
- 16: Anfangsabschnitt
- 17: Sollbruchstelle
- 18: Griffsteg
- 19: Ausformungen
- 20: Anformung
- 21: Einbuchtung
- 22: Abstand

## Patentansprüche

1. Zellkultureinsatz
mit einem hohlzylindrischen Gehäuse (8) mit einer stirnseitigen oberen, durch einen Öffnungsrand (9) begrenzten Öffnung und einem stirnseitigen unteren, als Membran ausgeführten Boden (10),
mit am bodenseitigen Rand des Gehäuses (8) und/oder am Boden (10) angeordneten, nach unten abragenden Stützfüßchen (11), mit denen das Gehäuse (8) auf einer Unterlage mit geringem, gleichmäßigem Abstand des Bodens (10) von der Unterlage kippsicher abstellbar ist, und
mit mindestens einem am Öffnungsrand (9) vom Gehäuse (8) nach außen abragenden Tragarm (12), der auf einem Rand (5') eines Wells (3) einer Wellplatte (2) auflegbar ist,
**dadurch gekennzeichnet,**
**dass** der Tragarm (12) als Rand-Einhängeelement ausgeführt ist und dazu einen radial zum Gehäuse (8) verlaufenden Auflageabschnitt (13) und einen vom Auflageabschnitt (13) nach unten in Richtung des Bodens (10) des Gehäuses (8) verlaufenden, vom Gehäuse (8) beabstandeten Einhängeabschnitt (14) aufweist.

2. Zellkultureinsatz nach Anspruch 1, **dadurch gekennzeichnet, dass** der lichte Abstand des Einhängeabschnittes (14) von dem Gehäuse (8) größer ist als die Dicke des Randes (5') eines Wells (3), so dass sich für den im Well (3) eingesetzten Zellkultureinsatz (7) ein Spielsitz ergibt.

3. Zellkultureinsatz nach Anspruch 1 oder 2, **dadurch gekennzeichnet, dass** der Tragarm (12) einen vom Gehäuse (8) aus nach oben verlaufenden Anfangsabschnitt (16) aufweist, an den sich der Auflageabschnitt (13) anschließt, so dass bei horizontal ausgerichtetem Gehäuse (8) der untere Rand des Auflageabschnitts (13) höher liegt als der Öffnungsrand (9).

4. Zellkultureinsatz nach einem der vorhergehenden Ansprüche, **dadurch gekennzeichnet, dass** der Tragarm (12) gehäusenah, vorzugsweise im Anfangsabschnitt (16) oder Auflageabschnitt (13), eine Sollbruchstelle (17) aufweist, an der der Tragarm (12) vom Gehäuse (8) abbrechbar ist, wobei die Sollbruchstelle (17) vorzugsweise kreisbogenförmig ausgeführt ist.

5. Zellkultureinsatz nach einem der vorhergehenden Ansprüche, **dadurch gekennzeichnet, dass** der Einhängeabschnitt (14) des Tragarms (12) nach außen verlängert als Griffsteg (18) für ein Greifwerkzeug, insbesondere für eine Pinzette, ausgeführt ist, wobei, vorzugsweise, beidseitig am Griffsteg (18) erhabene Anformungen (20) angeordnet sind, die die Schenkel der Pinzette halten und führen.

6. Zellkultureinsatz nach einem der vorhergehenden Ansprüche, **dadurch gekennzeichnet, dass** am Gehäuse (8) genau ein Tragarm (12) vorgesehen ist.

7. Zellkultureinsatz nach einem der vorhergehenden Ansprüche, **dadurch gekennzeichnet, dass** genau drei auf dem Umfang des Gehäuses (8) gleich beabstandete Stützfüßchen (11) vorgesehen sind.

8. Zellkultureinsatz nach einem der vorhergehenden Ansprüche, **dadurch gekennzeichnet, dass** die Stützfüßchen (11) vom Gehäuse (8) auch nach außen abragen.

9. Zellkultureinsatz nach Anspruch 8, **dadurch gekennzeichnet, dass** zwei Stützfüßchen (11) auf dem Umfang des Gehäuses (8) mit gleichem seitlichen Abstand zum Tragarm (12) angeordnet sind.

10. Zellkultureinsatz nach Anspruch 8, **dadurch gekennzeichnet, dass** die Anordnung des Einhängeabschnittes (14) des Tragarms (12) einerseits und der dem Tragarm (12) benachbarten Stützfüßchen (11) andererseits so gewählt ist, dass ein im Well (3) eingehängter Zellkultureinsatz (7) gegenüber dem Well (3) um nicht mehr als 10° geneigt angeordnet ist.

11. Zellkultureinsatz nach einem der vorhergehenden Ansprüche, **dadurch gekennzeichnet, dass** am Außenumfang des Gehäuses (8) gegenüber dem Tragarm (12) eine Einbuchtung (21) ausgeformt ist, die sich am Außenumgang symmetrisch zum Tragarm (12) und längs des Gehäuses (8) erstreckt, angrenzend zum Boden (10) ausgeformt ist und, vorzugsweise, mindestens 50% der Außenhöhe des Zellkultureinsatzes (7) einnimmt.

12. Vorrichtung zum Kultivieren von Zellen
mit einer Wellplatte (2), in der mehrere Wells (3) zur Aufnahme von Flüssigkeit ausgebildet sind,
wobei jedes Well (3) einen Boden (6) und eine davon aufragende, umlaufende Seitenwand (5) aufweist, wobei die Seitenwand (5) einen diese oben abschließenden Rand (5') aufweist, und
wobei jedes Well (3) in einer Ebene parallel zum Boden (6) bestimmte laterale Innenabmessungen, insbesondere einen bestimmten Innendurchmesser, und in Richtung senkrecht zum Boden (6) eine bestimmte Innenhöhe aufweist, und
mit mindestens einem Zellkultureinsatz (7) nach einem der Ansprüche 1 bis 6, der eine bestimmte Außenhöhe aufweist,
**dadurch gekennzeichnet,**
**dass** der Zellkultureinsatz (7) mit seinem Tragarm (12) am Rand (5') des Wells (3) einhängbar ist und
**dass** die Außenhöhe des Zellkultureinsatzes (7) geringer ist als die Innenhöhe des Wells (3), so dass sich bei eingehängtem Zellkultureinsatz (7) die Stützfüßchen (11) in einem Abstand vom Boden (6) des Wells (3) befinden.

13. Vorrichtung nach Anspruch 12, **dadurch gekennzeichnet, dass** der Zellkultureinsatz (7) bestimmte laterale Außenabmessungen aufweist und dass die lateralen Außenabmessungen des Zellkultureinsatzes (7) ohne Berücksichtigung des mindestens einen Tragarmes (12) geringer sind als die lateralen Innenabmessungen des Wells (3), und zwar dergestalt, dass das Gehäuse (8) des Zellkultureinsatzes (7) auf der dem eingehängten Tragarm (12) gegenüberliegenden Seite einen wesentlich größeren Abstand von der Seitenwand (5) des Wells (3) aufweist als auf der dem Tragarm (12) zugewandten Seite des Gehäuses (8).

14. Vorrichtung nach Anspruch 12 oder 13, **dadurch gekennzeichnet,**
**dass** die lateralen Außenabmessungen des Zellkultureinsatzes (7) mit Berücksichtigung des mindestens einen Tragarms (12) geringer sind als die lateralen Innenabmessungen des Wells (3) und
**dass** der Zellkultureinsatz (7) mitsamt dem mindestens einen Tragarm (12) auf dem Boden (6) des Wells (3) abstellbar ist,
wobei, vorzugsweise, das Gehäuse (8) des auf dem Boden (6) des Wells (3) abgestellten Zellkultureinsatzes (7) auf der dem Tragarm (12) zugewandten Seite des Gehäuses (8) einen wesentlich größeren Abstand von der Seitenwand (5) des Wells (3) aufweist als auf der vom Tragarm (12) abgewandten Seite des Gehäuses (8).

15. Vorrichtung nach einem der Ansprüche 12 bis 14, **dadurch gekennzeichnet, dass** die laterale geometrische Form des Wells (3) asymmetrisch relativ zur lateralen geometrischen Form des Gehäuses (8) des Zellkultureinsatzes (7) ausgebildet ist.

## Claims

1. Cell culture insert
having a hollow cylindrical housing (8) with an upper end-face opening bordered by an edge (9) of the opening and a lower end-face bottom (10) embodied as a membrane,
having supporting feet (11) arranged on the bottom edge of the housing (8) and/or on the bottom (10) and protruding downward, with which the housing (8) can be placed on a substrate in a tilt-proof manner at a small uniform distance of the bottom (10) from the substrate, and
having at least one carrying arm (12), which protrudes outward from the housing (8) on the edge (9) of the opening and which can be deposited on an edge (5') of a well (3) of a well plate (2),
**characterized in that**
the carrying arm (12) is embodied as an edge suspension element and for this purpose has a supporting section (13) running radially to the housing (8) and a suspension section (14) spaced from the housing (8), running down from the supporting section (13) in the direction of the bottom (10) of the housing (8).

2. Cell culture insert according to claim 1,
**characterized in that**
the inside clearance of the suspension section (14) from the housing (8) is greater than the thickness of the edge (5') of a well (3), thereby resulting in a clearance fit for the cell culture insert (7) inserted into the well (3).

3. Cell culture insert according to claim 1 or 2,
**characterized in that**
the carrying arm (12) has an initial section (16) running upward from the housing (8) and followed by the supporting section (13), so that when the housing (8) is oriented horizontally, the lower edge of the supporting section (13) is higher than the edge (9) of the opening.

4. Cell culture insert according to any one of the preceding claims, **characterized in that**
the carrying arm (12) has a predetermined breaking point (17) near the housing (8), preferably in the initial section (16) or in the supporting section (13), so that the carrying arm (12) can be broken off from the housing (8) at this predetermined breaking point, wherein the predetermined breaking point (17) is preferably embodied as an arc of a circle.

5. Cell culture insert according to any one of the preceding claims, **characterized in that**
the suspension section (14) of the carrying arm (12), lengthened outward, is embodied as a grip bar (18) for a gripping tool, in particular for tweezers, wherein, preferably, elevated molded parts (20) are arranged on both sides of the grip bar (18), holding and guiding the legs of the tweezers.

6. Cell culture insert according to any one of the preceding claims, **characterized in that**
exactly one carrying arm (12) is provided on the housing (8).

7. Cell culture insert according to any one of the preceding claims, **characterized in that**
exactly three supporting feet (11) spaced equal distances apart on the circumference of the housing (8) are provided.

8. Cell culture insert according to any one of the preceding claims, **characterized in that**
the supporting feet (11) also protrude outward from the housing (8).

9. Cell culture insert according to claim 8, **characterized in that**
two supporting feet (11) are arranged on the circumference of the housing (8) with the same lateral distance from the carrying arm (12).

10. Cell culture insert according to claim 8, **characterized in that**
the arrangement of the suspension section (14) of the carrying arm (12), on the one hand, and of the supporting feet (11) adjacent to the carrying arm (12), on the other hand, is selected so that a cell culture insert (7) suspended in the well (3) is arranged at an inclination of no more than 10° with respect to the well (3).

11. Cell culture insert according to any one of the preceding claims, **characterized in that**
an indentation (21) is formed on the outside circumference of the housing (8) vis-à-vis the carrying arm (12), extending along the outside circumference symmetrically with the carrying arm (12) and along the housing (8), formed adjacent to the bottom (10) and preferably taking up at least 50% of the outside height of the cell culture insert (7).

12. Device for culturing cells
comprising a well plate (2) in which a plurality of wells (3) is formed to hold liquid,
wherein each well (3) has a bottom (6) and has a peripheral side wall (5) protruding therefrom, wherein the side wall (5) has an edge (5') terminating the side wall (5) at the top, and
wherein each well (3) has certain lateral inside dimensions in a plane parallel to the bottom (6), in particular a certain inside diameter, and has a certain inside height in the direction perpendicular to the bottom (6), and
having at least one cell culture insert (7) according to any one of claims 1 through 6, of a certain outside height,
**characterized in that**
the cell culture insert (7) can be suspended with its carrying arm (12) on the edge (5') of the well (3), and
the outside height of the cell culture insert (7) is less than the inside height of the well (3), so that with the cell culture insert (7) suspended, the supporting feet (11) are located at a distance from the bottom (6) of the well (3).

13. Device according to claim 12,
**characterized in that**
the cell culture insert (7) has certain lateral outside dimensions, and the lateral outside dimensions of the cell culture insert (7) are smaller than the lateral inside dimensions of the well (3), not taking into account the at least one carrying arm (12), such that the housing (8) of the cell culture insert (7) has a much greater distance from the side wall (5) of the well (3) on the side opposite the suspended carrying arm (12) than on the side of the housing (8) facing the carrying arm (12).

14. Device according to claim 12 or 13,
**characterized in that**
the lateral outside dimensions of the cell culture insert (7) are smaller than the lateral inside dimensions of the well (3), taking into account the at least one carrying arm (12), and
the cell culture insert (7) together with the at least one carrying arm (12) can be deposited on the bottom (6) of the well (3),
wherein, preferably, the housing (8) of the cell culture insert (7) deposited on the bottom (6) of the well (3) has a much greater distance from the side wall (5) of the well (3) on the side of the housing (8) facing the carrying arm (12) than on the side of the housing (8) facing away from the carrying arm (12).

15. Device according to any one of claims 12 through 14,
**characterized in that**
the lateral geometric shape of the well (3) is designed to be asymmetrical relative to the lateral geometric shape of the housing (8) of the cell culture insert (7).

## Revendications

1. Insert de culture cellulaire
avec un boîtier cylindrique creux (8) avec une ouverture supérieure sur la face frontale limitée par un bord d'ouverture (9) et un fond inférieur (10) sur la face frontale conçu comme une membrane,
avec des pieds d'appui (11) faisant saillie vers le bas, disposés sur le bord inférieur du boîtier (8) et/ou sur le fond (10), avec lesquels le boîtier (8) peut être placé sur un socle à une faible distance uniforme du fonde (10) par rapport au socle de manière à ne pas pouvoir basculer, et
avec au moins un bras de support (12) qui fait saillie vers l'extérieur du boîtier (8) au niveau du bord de l'ouverture (9) et qui peut être placé sur un bord (5') d'une ondulation (3) d'une tôle ondulée (2),
**caractérisé en ce que,**
le bras de support (12) est conçu comme un élément de suspension de bord et comporte à cet effet une section de support (13) s'étendant radialement par rapport au boîtier (8) et une section de suspension (14) s'étendant à partir de la section de support (13) vers le bas en direction du fond (10) du boîtier (8) et à distance du boîtier (8).

2. Insert de culture cellulaire selon la revendication 1, **caractérisé en ce que** la distance libre de la section de suspension (14) par rapport au boîtier (8) est supérieure à l'épaisseur du bord (5') d'une ondulation (3), de sorte qu'un ajustement avec jeu est obtenu pour l'insert de culture cellulaire (7) inséré dans la ondulation (3).

3. Insert de culture cellulaire selon la revendication 1 ou 2, **caractérisé en ce que** le bras de support (12) comporte une section initiale (16) qui s'étend vers le haut depuis le boîtier (8) et à laquelle se raccorde la section de support (13), de sorte que lorsque le boîtier (8) est orienté horizontalement, le bord inférieur de la section de support (13) est plus haut que le bord d'ouverture (9).

4. Insert de culture cellulaire selon l'une des revendications précédentes, **caractérisé en ce que**
le bras de support (12) comporte un point de rupture prédéterminé (17) à proximité du boîtier, de préférence dans la section initiale (16) ou la section de support (13), auquel le bras de support (12) peut être cassé du boîtier (8), le point de rupture prédéterminé (17) étant de préférence conçu en forme d'arc de cercle.

5. Insert de culture cellulaire selon l'une des revendications précédentes, **caractérisé en ce que**
la section de suspension (14) du bras de support (12), prolongée vers l'extérieur, est conçue comme une bande de préhension (18) pour un outil de préhension, en particulier pour une pincette, des parties (20) surélevées, formées d'un seul tenant, étant disposées de préférence des deux côtés de la bande de préhension (18), qui maintiennent et guident les branches de la pincette.

6. Insert de culture cellulaire selon l'une des revendications précédentes, **caractérisé en ce qu'**exactement un bras de support (12) est prévu sur le boîtier (8).

7. Insert de culture cellulaire selon l'une des revendications précédentes, **caractérisé en ce qu'**il est prévu exactement trois pieds d'appui (11) équidistants sur la circonférence du boîtier (8).

8. Insert de culture cellulaire selon l'une des revendications précédentes, **caractérisé en ce que**
les pieds d'appui (11) font également saillie vers l'extérieur du boîtier (8).

9. Insert de culture cellulaire selon la revendication 8, **caractérisé en ce que** deux pieds d'appui (11) sont disposés sur la circonférence du boîtier (8) à la même distance latérale du bras de support (12).

10. Insert de culture cellulaire selon la revendication 8, **caractérisé en ce que** la disposition de la section de suspension (14) du bras de support (12) d'une part et des pieds d'appui (11) adjacents au bras de support (12) d'autre part est choisie de telle sorte qu'un insert de culture cellulaire (7) suspendu dans la ondulation (3) est incliné de 10° au maximum par rapport à la ondulation (3).

11. Insert de culture cellulaire selon l'une des revendications précédentes, **caractérisé en ce que**
sur la circonférence extérieure du boîtier (8), à l'opposé du bras de support (12), est formé un renfoncement (21) qui s'étend sur la circonférence extérieure symétriquement au bras de support (12) et le long du boîtier (8), qui est formé à proximité du fond (10) et qui occupe de préférence au moins 50 % de la hauteur extérieure de l'insert de culture cellulaire (7).

12. Dispositif pour la culture des cellules
ayant une tôle ondulée (2) dans laquelle une pluralité de ondulations (3) sont formés pour recevoir du liquide,
dans laquelle chaque ondulation (3) a un fond (6) et une paroi latérale périphérique (5) qui en fait saillie, la paroi latérale (5) ayant un bord (5') qui la ferme en haut, et chaque ondulation (3) ayant, dans un plan parallèle au fond (6), certaines dimensions intérieures latérales, en particulier un certain diamètre intérieur, et une certaine hauteur intérieure dans la direction perpendiculaire au fond (6), et
avec au moins un insert de culture cellulaire (7) selon l'une des revendications 1 à 6, qui a une certaine hauteur externe,
**caractérisé en ce que,**
l'insert de culture cellulaire (7) peut être suspendu avec son bras de support (12) au bord (5') de la ondulation (3), et
**en ce que** la hauteur externe de l'insert de culture cellulaire (7) est inférieure à la hauteur interne de la ondulation (3), de sorte que, lorsque l'insert de culture cellulaire (7) est inséré, les pieds d'appui (11) sont à une distance du fond (6) de la ondulation (3).

13. Dispositif selon la revendication 12, **caractérisé en ce que** l'insert de culture cellulaire (7) présente certaines dimensions extérieures latérales et que les dimensions extérieures latérales de l'insert de culture cellulaire (7) sont inférieures aux dimensions intérieures latérales de la ondulation (3) sans tenir compte du au moins un bras de support (12), de telle sorte que le boîtier (8) de l'insert de culture cellulaire (7) présente une distance sensiblement plus grande par rapport à la paroi latérale (5) du puits (3) du côté opposé au bras de support (12) inséré que du côté du boîtier (8) faisant face au bras de support (12).

14. Dispositif selon la revendication 12 ou 13, **caractérisé en ce que** en ce que les dimensions extérieures latérales de l'insert de culture cellulaire (7), compte tenu du bras de support (12) au moins, sont inférieures aux dimensions intérieures latérales du puits (3), et
que l'insert de culture cellulaire (7) ainsi qu'au moins un bras de support (12) peuvent être placés sur le fond (6) du puits (3),
dans lequel, de préférence, le logement (8) de l'insert de culture cellulaire (7) placé sur le fond (6) du puits (3) présente une distance sensiblement plus grande par rapport à la paroi latérale (5) de la ondulation (3) sur le côté du boîtier (8) faisant face au bras de support (12) que sur le côté du boîtier (8) opposé au bras de support (12).

15. Dispositif selon l'une des revendications 12 à 14, **caractérisé en ce que** la forme géométrique latérale de la ondulation (3) est asymétrique par rapport à la forme géométrique latérale du boîtier (8) de l'insert de culture cellulaire (7).
